# EUROPEAN PATENT APPLICATION

(11) **EP 4 454 665 A1**
(43) Date of publication of application: **30.10.2024**
(21) Application number: 22911683.5
(22) Date of filing: 02.12.2022
(51) Int. Cl.: A61K 48/00, A61K 31/713, A61P 9/10

(54) **COMPOSITION FOR PREVENTING OR TREATING ISCHEMIA-REPERFUSION INJURY AND USE THEREOF**

(30) Priority: 22.12.2021 KR 20210185376
(71) Applicant: NA Vaccine Institute, Seoul 05854 (KR)
(72) Inventor: KIM, Dong Ho, Seongnam-si, Gyeonggi-do 13640 (KR); KANG, Myung Soo, Seoul 05702 (KR); CHA, Seung Bin, Seoul 06584 (KR); LEE, Seung Hwan, Gwangju-si, Gyeonggi-do 12787 (KR)
(74) Representative: Patentanwälte Olbricht Buchhold Keulertz
(86) International application number: PCT/KR2022/019524
(87) International publication number: WO 2023/121050

(57) **Abstract**

Provided are a composition for preventing or treating an ischemia-reperfusion injury and use thereof.

## Description

### Technical Field

The present disclosure relates to a composition for preventing or treating an ischemia-reperfusion injury and use thereof.

### Background Art

ischemia-reperfusion injury is a common clinical issue. The ischemia-reperfusion injury may be caused by spontaneous events, such as heart attack and stroke, or may be artificially induced by surgery and organ transplantation. Standard declotting and antiplatelet treatment as a part of reperfusion procedures are intended to save the life of a patient, but are not enough to address tissue weakness and/or organ damage caused by ischemia-reperfusion. The ischemia-reperfusion injury is the main cause of death in individuals after such treatments.

Therefore, there is a need for effective therapies to treat the ischemia-reperfusion injury.

### Disclosure

### Technical Problem

An aspect provides a composition for preventing or treating an ischemia-reperfusion injury in a subject, the composition including an isolated double-stranded RNA molecule with 3'-overhangs.

Another aspect provides a method of preventing or treating an ischemia-reperfusion injury in a subject, the method including administering a therapeutically effective amount of a double-stranded RNA molecule to a subject.

### Technical Solution

In the present specification, unless otherwise stated regarding the location of nucleotide sequences, nucleotide sequences are linked or located in the direction from a 5'-end to a 3'-end.

In the present specification, the term "vector" refers to any nucleic acid capable of delivering a gene or a nucleic acid fragment including DNA, into a cell. The vector may be, for example, replicated in a cell. The vector may be a virus, a bacteriophage, a provirus, a plasmid, a phagemid, a transposon, and an artificial chromosome.

In the present specification, regarding polypeptides or polynucleotides, the term "identity" refers to the relationship between sequences of two or more polypeptides or polynucleotides as determined by comparing the sequences. The identity represents a degree of sequence relatedness determined by the number of matches between strings of two or more amino acid residues or nucleotide residues. The identity of related polypeptides or polynucleotides may be calculated by known methods. "% identity," as applied to polypeptides or polynucleotides, is defined as the percentage of residues in candidate amino acid sequences or nucleotide sequences that are identical to residues in a second sequence, after aligning and, if necessary, introducing gaps, to achieve maximum percent identity with the second sequence. Methods and programs for the alignment are known. Such programs may be, for example, BLAST, Smith-Waterman algorithm, or Needleman-Wunsch algorithm.

In the present specification, the term "operatively linked" refers that nucleotide sequences are linked on a single nucleic acid fragment such that one function is affected by another. For example, a promoter may be operatively linked to a coding sequence (e.g., ORF) when the promoter can affect expression of the coding sequence (i.e., a case where the coding sequence is under the transcriptional control of the promoter). The coding sequence may be operatively linked to a regulatory sequence in either the sense or antisense direction.

In the present specification, the term "ischemia-reperfusion injury (IR injury)" refers to tissue damage caused by ischemia, reperfusion, or reperfusion after ischemia. Therefore, the term "IR damage" may include ischemia-induced injury, reperfusion injury, or injury by reperfusion after ischemia. As used in the present specification, "an event that causes an IR injury" may include ischemia, reperfusion, thromboembolism, vasoconstriction, cardiac standstill, myocardial infarction, stroke, or surgeries (e.g., organ transplantation, heart surgery, etc.) in which blood flow to tissues is blocked or reduced, and the like. Myocardial infarction (Ml) is a type of cardiac ischemia that can cause an IR injury.

An aspect provides a composition for preventing or treating an ischemia-reperfusion injury in a subject, the composition including an isolated double-stranded RNA molecule, wherein the isolated double-stranded RNA molecule includes a first strand including a 5'-end, a 3'-end, and a region having complementarity with a second strand, the second strand including a 5'-end, a 3'-end, and a region having complementarity with the first strand, a first overhang having one or more consecutive ribonucleotides and being located at the 3'-end of the first strand, and a second overhang having one or more consecutive ribonucleotides and being located at the 3'-end of the second strand.

The isolated double-stranded RNA molecule may serve as a TLR3 ligand. The isolated double-stranded RNA molecule may be a TLR3 agonist. When the isolated double-stranded RNA molecule is administered to a subject, the immune response of the subject may be promoted.

The isolated double-stranded RNA molecule may be recombinant. The isolated double-stranded RNA molecule may include not only naturally occurring ribonucleotides, but also modified ribonucleotides. The modified ribonucleotides may be known in the art.

In the first strand and the second strand, the regions having complementarity may independently have a length of 100 nt to 1,700 nt, for example, 106 nt to 1,648 nt, 200 nt to 1,500 nt, 300 nt to 1,000 nt, 400 nt to 900 nt, 106 nt to 500 nt, 200 nt to 500 nt, or 200 nt to 400 nt. In the first strand and the second strand, the region having complementarity with the second strand may have complementarity with the region having complementarity to the first strand.

In the first strand and the second strand, the first overhang and the second overhang may independently have a length of 2 nt or more, 3 nt or more, 4 nt or more, 5 nt or more, 6 nt or more, 7 nt or more, 8 nt or more, 9 nt or more, 10 nt or more, or 15 nt or more.

In the first strand and the second strand, the first overhang and the second overhang may not include sequences complementary to each other. The first overhang and the second overhang may not include sequences complementary to each other, and thus may not form a double strand.

In the first strand and the second strand, the 5'-ends may not independently have a 5'-cap.

In the first strand and the second strand, the 5'-ends may independently include triphosphate, diphosphate, or monophosphate.

In the first strand and the second strand, the 3'-ends may independently include phosphate.

In the first strand and the second strand, the 3'-ends may independently include a hydroxyl group.

In the first strand and the second strand, the regions having complementarity may not independently be a homopolynucleotide. The homopolynucleotide may be polycytidylate, polyuridylate, polyadenylate, polyinosinylate, or polyguanylate. The isolated double-stranded RNA molecule may not be poly(I:C), poly(I:C)-L-lysine (poly (I:C)-L), poly(I:C)-L-lysine-methylcellulose (poly (I:C)-LC), or poly(I:C12U).

The isolated double-stranded RNA molecule may not have RNAi or antisense inhibitory activity, may have minimal RNAi or antisense inhibitory activity.

The isolated double-stranded RNA molecule may not have an intramolecular stem-and-loop structure. The isolated double-stranded RNA molecule may not have an intramolecular stem-and-loop structure in a double-stranded region formed between the region having complementarity with the second strand and the region having complementarity with the first strand.

In the first strand and the second strand, the first overhang and the second overhang may independently have the same length or different lengths. The first overhang and the second overhang may independently have 1 to 75 ribonucleotides, for example, 2 to 50 ribonucleotides, 3 to 50 ribonucleotides, 4 to 50 ribonucleotides, 5 to 50 ribonucleotides, 1 to 30 ribonucleotides, 2 to 30 ribonucleotides, 3 to 30 ribonucleotides, 4 to 30 ribonucleotides, or 5 to 30 ribonucleotides.

In the first strand and the second strand, the first overhang and the second overhang may independently have a length of 1, 2, 3, 4, 5, 6, 7, 8, 9, or 10 ribonucleotides.

In the first strand and the second strand, the regions having complementarity may have 75 % or more, 80 % or more, 85 % or more, 90 % or more, 95 % or more, or 100 % nucleotide sequence complementarity between the nucleotides of the first strand and the nucleotides of the second strand. The regions having complementarity may have complementary nucleotides at positions where all nucleotides correspond to the nucleotide sequences of the first strand and the second strand.

The isolated double-stranded RNA molecule may not have an intramolecular single-stranded region in a double-stranded region formed between the region having complementarity with the second strand and the region having complementarity with the first strand. The isolated double-stranded RNA molecule may not have an intramolecular nick.

In the first strand and the second strand, the regions having complementarity may have an intermolecular Watson-Crick hydrogen bond.

In the first strand and the second strand, the regions having complementarity may not independently include complementary sequences to the nucleotide sequences of the human genome. The complementary sequences to the nucleotide sequences of the human genome may be 20 or more consecutive nucleotide sequences.

In the first strand and the second strand, the regions having complementarity may not independently include 5, 6, 7, 8, 9, 10, 11, 12, 13, 14, 15, 16, 17, 18, 19, 20, 21, 22, 23, 24, 25, 26, 27, 28, 29, or 30 consecutive nucleotide sequences with respect to the nucleotide sequences of the human genome, or may not include complementary sequences to the entire nucleotide sequences of the region having complementarity.

In the first strand and the second strand, the regions having complementarity may independently be derived from a non-human subject. The subject may of viral, bacterial, or plant origin. The virus may be Sacbrood virus (SBC). The isolated double-stranded RNA molecule may be derived from a VP1 protein gene of the SBC.

The isolated double-stranded RNA molecule may include the first strand including the 5'-end, the 3'-end, and the region having complementarity with the second strand, the second strand including the 5'-end, the 3'-end, and the region having complementarity with the first strand, the first overhang having one or more consecutive ribonucleotides and being located at the 3'-end of the first strand, and the second overhang having one or more consecutive ribonucleotides and being located at the 3'-end of the second strand,

wherein the region having complementarity with the second strand and the region having complementarity with the first strand may each have a length of 100 nt to 1,700 nt, 106 nt to 1,648 nt, 200 nt to 1,500 nt, 300 nt to 1,000 nt, 400 nt to 900 nt, 106 nt to 500 nt, 200 nt to 500 nt, or 200 nt to 400 nt,
in the first strand and the second strand, the first overhang and the second overhang may independently have a length of 1 nt or more, 2 nt or more, 3 nt or more, 4 nt or more, 5 nt or more, 6 nt or more, 7 nt or more, 8 nt or more, 9 nt or more, 10 nt or more, or 15 nt or more, and the ends may include a UAUAG sequence,
in the first strand and the second strand, the 5'-ends may not independently have a 5'-cap,
in the first strand and the second strand, the 5'-ends may independently include triphosphate, diphosphate, or monophosphate,
in the first strand and the second strand, the 3'-ends may independently include
a hydroxyl group, and
in the first strand and the second strand, the region having complementarity with the second strand and the region having complementarity with the first strand may be derived from the VP1 protein gene of the SBC.

The composition may include a pharmaceutically acceptable carrier. In the present specification, the term "carrier" may be a compound that promotes delivery to and/or complexation with another compound. The other compound may be a cargo to be delivered. The carrier may be a polymeric carrier. The carrier may be linked to the cargo via a covalent or non-covalent interaction. The carrier may deliver a nucleic acid, such as RNA, to a target cell. The carrier may be a cationic component. The cationic component refers to a positively charged molecule at pH of, for example, 1 to 9, 5 to 9, 5 to 8, or 5 to 7, or at physiological pH. The physiological pH may be a pH of 7.3 to 7.4. The cationic component may be a positively charged cationic peptide or polypeptide under physiological conditions. The cationic peptide or polypeptide may include one or more positively charged amino acids, such as one or more Arg, His, Lys, or Orn or a combination thereof.

The composition may also include a pharmaceutically acceptable excipient or diluent. The excipient may be a disintegrant, a binding agent, a glydent, a sweetening agent, or a thickener. The diluent may include an aqueous solution such as an aqueous buffer solution. In addition, the composition may further include an adjuvant to promote an immune response in the subject.

The composition may have an oral or parenteral dosage formulation. The formulation may be a liquid formulation such as an injection. The parenteral dosage formulation may be an intramuscular, subcutaneous, intradermal, nasal, or pulmonary dosage formulation.

The relative amounts of the active components included in the composition, i.e., the double-stranded RNA and other additional components, may vary depending on a subject to be treated, size and/or conditions of the subject, and a dosage route. For example, the composition may include the double-stranded RNA in an amount of 0.1 % to 100 %, for example, 0.5 % to 50 %, 1.0 % to 30 %, 5.0 % to 80 %, or 80 %(w/w) or more. The composition may be formulated in nanoparticles. The composition may be formulated in lipid nanoparticles (LNPs). For example, the composition may be formulated in a lipid polycation composite.

In the first strand and the second strand, the first overhang and the second overhang may independently be obtained by RNase digestion, e.g., RNase T1 digestion. In the first strand and the second strand, the first overhang and the second overhang may independently be obtained by hybridizing a pre-first strand with a pre-second strand, the pre-first strand having an extended nucleotide at the 3'-end relative to the first strand, and the pre-second strand having an extended nucleotide at the 3'-end relative to the second strand, and contacting a hybridized product with RNase T1 to digest ssRNA at the 3'-end. The RNase may be an endoribonuclease or exoribonuclease, which specifically cleaves the single-stranded RNA. The RNase T1 may be an endoribonuclease that specifically cleaves the single-stranded RNA at the G residue. The RNase T1 may cleave the phosphodiester bond between a 3'-guanyl residue and a 5'-OH residue of a neighboring nucleotide. The reaction product may be a polynucleotide having 3'-GMP and terminal 3'-GMP. The reaction product may not have a single-stranded region other than the 3'-overhang region. The reaction product may not have a nick.

In the first strand and the second strand, the first overhang and the second overhang may independently have GMP at the 3'-end. In the first strand and the second strand, the first overhang and the second overhang may independently have the UAUAG sequence at the 3'-end.

In the first strand and the second strand, the regions having complementarity may independently have the UAUAG sequence at the 3'-end. The first strand and the second strand may have the nucleotide sequences of SEQ ID NOs: 1 and 2, respectively.

Another aspect provides a method of preventing or treating an ischemia-reperfusion injury in a subject, the method including administering a therapeutically effective amount of a double-stranded RNA molecule to a subject, wherein the double-stranded RNA molecule may include the first strand including the 5'-end, the 3'-end, and the region having complementarity with the second strand, the second strand including the 5'-end, the 3'-end, and the region having complementarity with the first strand, the first overhang having one or more consecutive ribonucleotides and being located at the 3'-end of the first strand, and the second overhang having one or more consecutive ribonucleotides and being located at the 3'-end of the second strand.

In the method, the double-stranded RNA molecule is the same as described herein. The method may include administering the composition to the subject.

In the method, the term "therapeutically effective amount" may be a minimum amount of an active component, e.g., the double-stranded RNA, required to prevent or treat the IR injury. The IR injury may include brain ischemia, ischemic brain injury, ischemic stroke (IS), hemorrhagic stroke, traumatic brain injury, or spinal cord injury.

The therapeutically effective amount may vary depending on the selected subject, gender, site and severity of IR injury, etc. The therapeutically effective amount may be, for example, 10 µg to 100 mg, 10 µg to 50 mg, 10 µg to 10 mg, 10 µg to 1 mg, or 10 ng to 10 mg, per body weight (kg).

The administration may be oral or parenteral administration. The administration may be, for example, intramuscular, subcutaneous, intradermal, nasal, or pulmonary administration. The administration may be by nasal instillation.

The administration may be performed once or multiple times. The administration may be, for example, performed twice or more, 3 or more times, 4 or more times, 5 or more times, or 6 or more times. The administration may be performed within 30 minutes or 1, 2, 3, 4, 5, 6, 12, or 24 hours after ischemia occurs.

### Advantageous Effects

The composition including an isolated double-stranded RNA molecule for preventing or treating an IR injury in a subject according to an aspect may be used to prevent or treat the IR injury in the subject.

By using the method for preventing or treating an IR injury in a subject according to an aspect, the method including administering a therapeutically effective amount of a double-stranded RNA molecule to a subject, the IR injury may be effectively prevented or treated in the subject.

### Description of Drawings

FIG. 1 schematically shows obtained double-stranded RNA.
FIG. 2A is a diagram showing an experimental procedure to confirm a therapeutic effect on stroke in a rat model with acute ischemic stroke according to administration of double-stranded RNA.
FIG. 2B is a diagram showing a relative volume of a cerebral infarction region in a rat model with acute ischemic stroke according to administration of double-stranded RNA.
FIG. 2C is a diagram showing results of measuring a mNSS value in a rat model with acute ischemic stroke according to administration of double-stranded RNA.
FIG. 2D is a diagram showing changes in body weight in a rat model with acute ischemic stroke according to administration of double-stranded RNA.
FIGS. 2E and 2F are photographs showing results of TTC staining on brain matrix slices of brains extracted from groups G1 and G3 of a rat model with acute ischemic stroke administered with double-stranded RNA.
FIG. 3A is a diagram showing an experimental procedure in which double-stranded RNA having 3'-overhangs is administered to a rat model with acute ischemic stroke, in two different dosages and at different times, to confirm a therapeutic effect thereof on stroke.
FIG. 3B is a diagram showing a volume percentage of cerebral infarction regions when double-stranded RNA with 3'-overhangs is administered to a rat model with acute ischemic stroke.
FIG. 3C is a diagram showing results of measuring mNSS values for a rat model with acute ischemic stroke.
FIGS. 3D, 3D, 3F, and 3G are photographsshowing results of TTC staining on brain matrix slices of brains extracted 7 days after ischemia from the groups G1, G2, G5, and G6 of a rat model with acute ischemic stroke administered with double-stranded RNA.
FIG. 4 is a diagram showing a survival rate of experimental animals according to a dosage of double-stranded RNA.
FIG. 5 is a diagram showing a total distance moved by a rat in an open field test.
FIG. 6 is a diagram showing results of Golgi-cox staining and Sholl analyses on brains of experimental animals according to a dosage of double-stranded RNA.
FIG. 7A is a diagram showing results of analyzing neurite outgrowth of pyramidal neurons according to Sholl analysis.
FIG. 7B is a diagram showing results of analyzing branch points of pyramidal neurons according to Sholl analysis.
FIG. 7C is a diagram showing results of analyzing endpoints of pyramidal neurons according to Sholl analysis.

### Best Mode

### Mode for Invention

Hereinafter, the present disclosure will be described in detail with reference to Examples below. However, these Examples are for illustrative purposes only, and the scope of the present disclosure is not intended to be limited by these Examples.

### Example1: Double-stranded RNA molecule with 3'-overhang and confirmation of therapeutic effect thereof on ischemia-reperfusion injury (IR injury)

In this example, a double-stranded RNA molecule having a 3'-overhang was prepared, and its effect in preventing or treating an IR injury was confirmed.

In this example, the double-stranded RNA molecule having a 3'-overhang has a structure in which the nucleotide sequences of SEQ ID NOs: 1 and 2 are linked by complementing binding.

### 1. Preparation of double-stranded RNA molecule with 3'-overhang

A DNA fragment was synthesized in which a sequence including a T7 promoter sequence (SEQ ID NO: 3) was linked to each 5'-end of the DNA sequence encoding the nucleotide sequences of SEQ ID NOs: 1 and 2. The DNA fragment is dsDNA formed by the nucleotide sequence of SEQ ID NO: 4 and the complementary sequence thereof. The DNA fragment was ligated into a PUC19 vector (Thermo Fisher, Cat SD0061) cleaved with Smal, to manufacture a recombinant vector. Next, the recombinant vector was introduced into *E*. coli DH5a by a transformation method. The recombinant *E*. *coli* thus obtained was cultured in a LB/amp medium to proliferate *E*. *coli* including the recombinant vector. The *E*. coli proliferated in the culture was isolated from the supernatant, and the recombinant vector was isolated from the *E*. *coli* by an alkaline lysis method (Qiagen midi prep kit). A template DNA for in vitro transcription (IVT) based on a T7 polymerase was amplified by PCR using the recombinant vector DNA as a template. Here, oligonucleotides having the nucleotide sequences of SEQ ID NOs: 5 and 6, which correspond to the sequences at both ends of the sequence to be amplified, were used as a primer set. PCR conditions were as follows. One cycle was performed at 95 °C for 5 minutes, and then a thermal cycle consisting of 95 °C for 30 seconds, 60 °C for 30 seconds, and 72 °C for 1 minute was repeated 35 times. Finally, the reaction product was maintained at 72 °C for 5 minutes, and the PCR reaction was terminated. Using the amplified DNA product as a template, IVT was performed by using a MEGAscript^{™} T7 transcription kit (Thermo Fisher, cat AMB13345). 10 µg linear template DNA, 75 mM NTP, 90 mM tris base, 90 mM boric acid, 2 mM EDTA, and 50 µl T7 polymerase were added to 1 ml of a reaction solution, and allowed for a reaction at 37 °C for 4 hours. After completion of the reaction, the mixture was heat treated at 80 °C for 20 minutes and cooled at room temperature for 30 minutes. By the IVT, a first strand and a second strand were created simultaneously from both strands, and then hybridized to form double-stranded RNA. The cooled reaction mixture was centrifuged at 4,000 rpm at 20 °C for 3 minutes by using a centrifuge, to remove the white precipitate and obtain a supernatant. DNAse I (1/6,500 dilution from stock) was added to the obtained supernatant, and allowed for a reaction at 37 °C for 13 hours. Afterwards, RNase T1 (1/20,000 dilution) was added to the reaction mixture, and allowed for a reaction at 37 °C for 2 hours. Afterwards, the reaction mixture was incubated at 80 °C for 10 minutes, and then cooled at room temperature for 30 minutes. Next, by using nucleic acid precipitation using isopropanol, a double-stranded RNA molecule having a 3'-overhang at each end was isolated from the reaction mixture.

The double-stranded RNA had phosphate at the 5'-end and a hydroxyl group at the 3'-end. The phosphate may be triphosphate, diphosphate, or monophosphate. The double-stranded RNA may not have a 5'-cap.

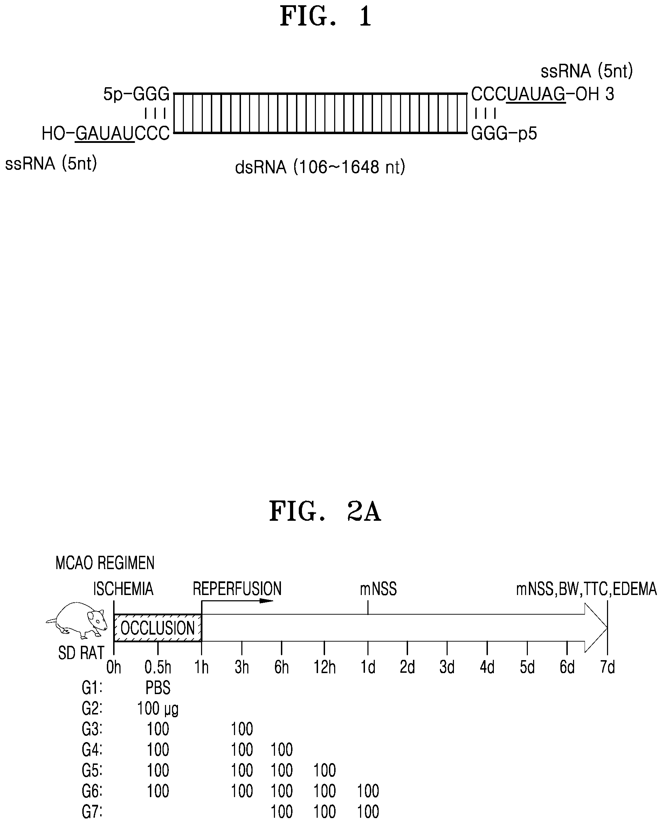

FIG. 1 schematically shows the obtained double-stranded RNA. In FIG. 1, the dsRNA portion is the complementary region and has a length of 424 nt.

### 2. Confirmation of prevention or treatment effect of double-stranded RNA molecule with 3'-overhang on IR injury

The double-stranded RNA obtained in Section 1 was administered to an IR injury animal model, and it was confirmed whether an IR injury was ameliorated.

In detail, ischemia or infarction was induced by middle cerebral artery occlusion (MCAO) for 60 minutes in 7-week-old male SD rats, and an IR injury was induced immediately thereafter by reperfusion or recanalization. Animals were separated into groups of four animals. Accordingly, acute stroke was induced in the animal models. Afterwards, 50 µL of double-stranded RNA solution (concentration: 2 g/µL) in PBS was instilled into the nasal cavity of the animal to administer 100 µg of double-stranded RNA. The control group used PBS (G1), and for the experimental groups, 100 µg of double-stranded RNA in PBS was instilled, from the onset of ischemia, once at 0.5 hours (G2), once at 0.5 hours and once 3 hours for a total of twice (G3), once at 0.5 hours, once at 3 hours, and once at 6 hours for a total of three times (G4), once at 0.5 hours, once at 3 hours, once at 6 hours, and once at 12 hours for a total of four times (G5), once at 0.5 hours, once at 3 hours, once at 6 hours, once at 12 hours, and once a day for a total of five times (G6), and once at 6 hours, once at 12 hours, and once a day for a total of three times (G7).

On Days 1 and 7 after stroke induction, mNSS, which is a behavioral indicator of stroke severity, and body weight were measured, and the volume of cerebral infarction area was measured by TTC staining of cerebral infarction sections. Detailed procedure is as follows.

### (1) Middle cerebral artery occlusion (MCAO) method

First, a rat weighing 258 g to 275 g was anesthetized by inhalation of isoflurane. The hair of a surgical area, the neck, was removed, and the hair removal site on the neck was disinfected with 70 % alcohol. A skin incision was made slightly to the left of the center. Then, the common carotid artery was identified by dissecting the muscle layer of the left three strands of the bronchus. The blood flow of the superior thyroid artery and occipital artery located on the left and right of the common carotid artery was blocked and cut off. The lower end of the common carotid artery and the internal carotid artery were clamped to block the blood flow. The upper part of the common carotid artery was ligated by using a surgical thread, and the middle part was loosely ligated. The middle part of the ligation was cut, and a filament was inserted into the blood vessel. The ligation in the middle part was tightly tied to prevent the filament from falling out. Then, ischemia was induced by inserting the intravascular filament into the middle cerebral artery. Here, a close attention was required to avoid entering to the pterygopalatine artery. When the ischemia state was confirmed upon the insertion of the filament, the skin was sutured. After 1 hour, the ischemia-induced animal was anesthetized by inhalation of isoflurane again for reperfusion. The lower end of the common carotid artery was clamped to block the blood flow, and then the filament was removed. After the filament was removed, the blood vessel was tied tightly to prevent blood leakage into the loosely ligated area. Reperfusion was performed by removing the clamp at the lower end of the common carotid artery. Here, it was checked whether the blood flow was flowing well or leaked. Following the reperfusion, intranasal administration was performed 0.5 hours, 3 hours, 6 hours, 12 hours, 24 hours, 48 hours, 72 hours, 96 hours, 120 hours, and 144 hours after the ischemia occlusion, according to respective administration route and time. The skin was sutured by using sutures.

### (2) Behavior test

Behavioral tests were conducted on test animals based on a modified neurological severity score (mNSS), before ischemia, 24 hours after ischemia, and 7 days after ischemia.

### (3) Autopsy and measurement of necrosis area

After 7 days of ischemia, the animals were observed for symptoms, and the brains were removed, sliced at a thickness of 2 mm on a brain matrix, and then stained with TTC. The extracted forebrain was cut coronally at about 3 mm intervals beginning from the front of the frontal lobe to produce 8 sections. Then, these cerebral tissue sections were soaked in a 2 % triphenyltetrazolium chloride (TTC) solution for 30 minutes at 37 °C under light-blocking conditions for staining while being turned over every 10 minutes. Afterwards, each section was placed on a surgical microscope, and the infarcted area was observed. The sections were washed with distilled water and fixed in a 10% formalin solution. To measure the infarction extent by TTC staining, the area of the cerebral infarction extent and its percentage (%), relative to the total area in each section, were calculated by using a computer image analysis system (IBAS 2000, Kontron, West Germany) according to an equation below: Infarct volume (mm3) = uninfarcted hemishere - (infarcted hemishere - infarcted area).

The infarct volume percent was obtained by converting the infarcted volume as a percentage of the uninfarcted hemisphere volume. Here, edema was expressed as a ratio of edema in the infarcted hemisphere to edema in the non-infarcted hemisphere.

FIGS. 2A to 2D are diagrams showing a therapeutic effect on stroke in a rat model with acute ischemic stroke according to administration of double-stranded RNA.

FIG. 2A is a diagram showing an experimental procedure to confirm a therapeutic effect on stroke in a rat model with acute ischemic stroke according to administration of double-stranded RNA.

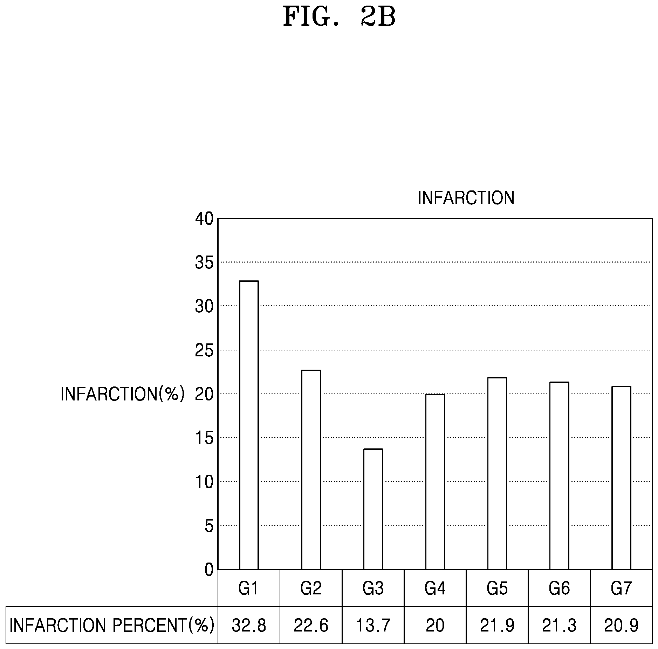

FIG. 2B is a diagram showing a relative volume of a cerebral infarction region in a rat model with acute ischemic stroke according to administration of double-stranded RNA. As shown in FIG. 2B, the double-stranded RNA administration groups G2, G3, G4, G5, G6, and G7 showed a lower infarction percentage compared to the control group G1.

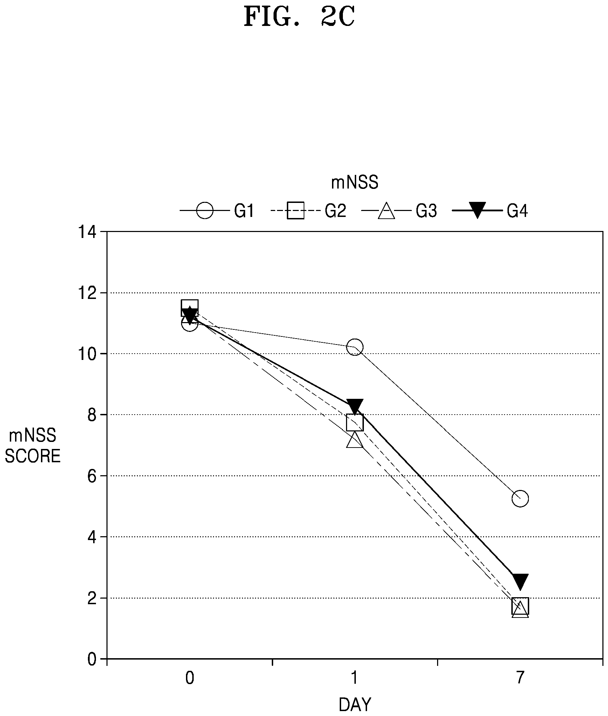

FIG. 2C is a diagram showing results of measuring a mNSS value in a rat model with acute ischemic stroke according to administration of double-stranded RNA. The mNSS value indicates the degree of aftereffects due to stroke, and the lower the value, the lower the aftereffects. As shown in FIG. 2C, the double-stranded RNA administration groups G1, G2, G3, and G4 showed lower mNSS values than the control group G1.

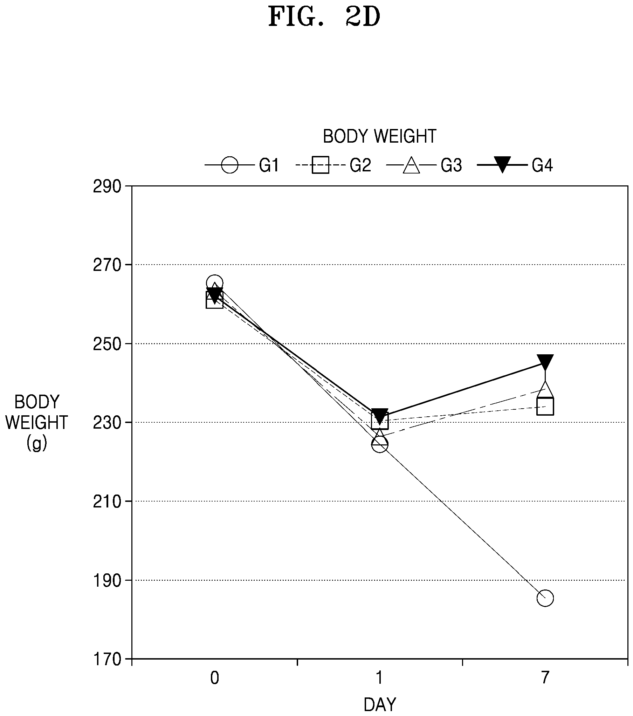

FIG. 2D is a diagram showing changes in body weight in a rat model with acute ischemic stroke according to administration of double-stranded RNA. As shown in FIG. 2D, the double-stranded RNA administration groups G1, G2, G3, and G4 increased body weight compared to the control group G1.

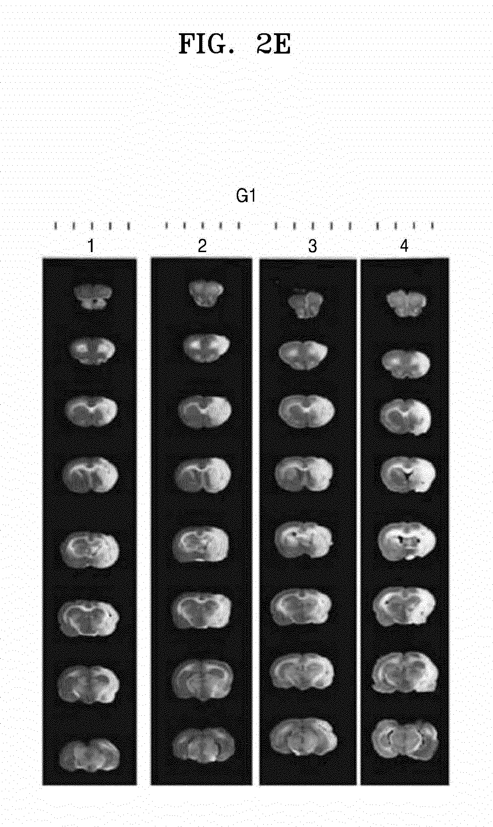

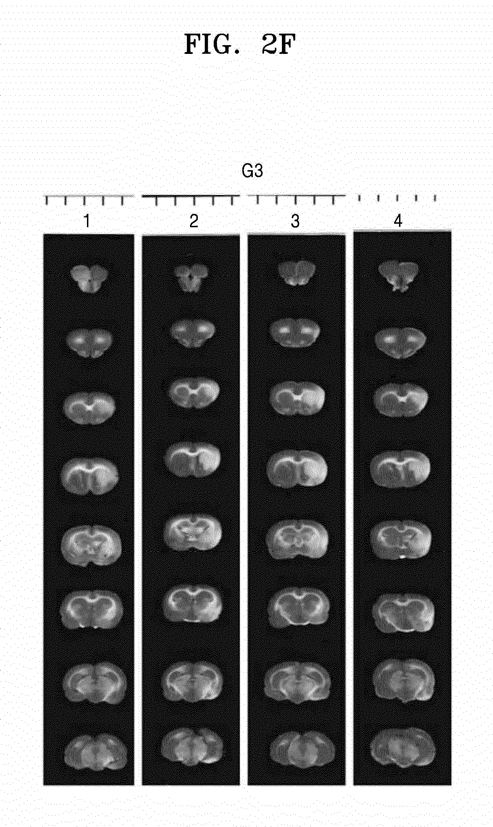

FIGS. 2E and 2F are photographs showing results of TTC staining on brain matrix slices of brains extracted from the control group G1, which uses PBS as an excipient and is not subjected to any drug treatment, and the group G3, which is a representative group among the groups treated with the drug of the present disclosure, among the rat models with acute ischemic stroke administered with double-stranded RNA, after 7days of ischemia. In FIGS. 2E and 2F, from top to bottom, the top slices of the brain matrix to the bottom slices of the brain matrix are shown. As shown in FIGS. 2E and 2F, the cerebral infarction occurred extensively in the hemibrain in the group G1, which is a negative control group treated with PBS, whereas the infarction area was significantly reduced in the group G3 which is treated with the drug. In FIGS. 2E and 2F, the cerebral infarction area is a white-stained area.

### 3. Confirmation of double-stranded RNA molecule with 3'-overhang in preventing or treating IR injury at different concentrations and times

In this section, the experiment was performed in the same manner as described in Section 2, except that the dosage and administration time were different. The double-stranded RNA was administered in dosages of 20 µg and 50 µg, and was administered multiple times at different times. The control group used PBS (G1), and for the experimental groups, 20 µg of the double-stranded RNA was administered twice in total, once at 0.5 hours and once at 3 hours (G2), once at 4.5 hours and once at 7 hours (G3), once at 6 hours and once at 8.5 hours (G4), and 50 µg of the double-stranded RNA was administered twice in total, once at 0.5 hours and once at 3 hours (G5), once at 4.5 hours and 7 hours (G6), and once at 6 hours and once at 8.5 hours (G7).

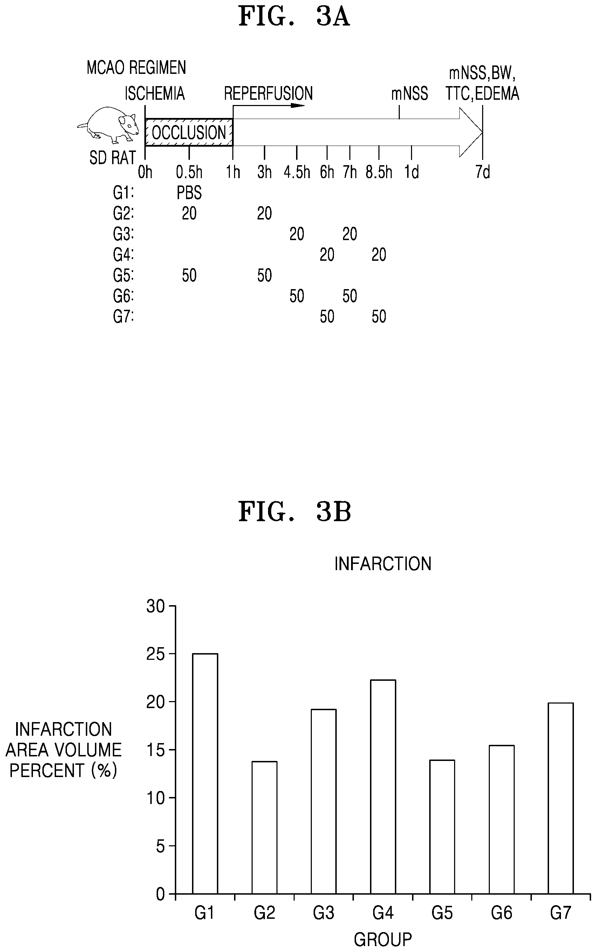

FIGS. 3A to 3G are diagrams showing the results of confirming the therapeutic effect on stroke after administering the double-stranded RNA having 3'-overhangs to the rat model with acute ischemic stroke in two different dosages and at different times.

FIG. 3A is a diagram showing an experimental procedure in which the double-stranded RNA having 3'-overhangs was administered to the rat model with acute ischemic stroke, in two different dosages and at different times, to confirm the therapeutic effect thereof on stroke.

FIG. 3B is a diagram showing a volume percentage of cerebral infarction regions when the double-stranded RNA with 3'-overhangs was administered to the rat model with acute ischemic stroke.

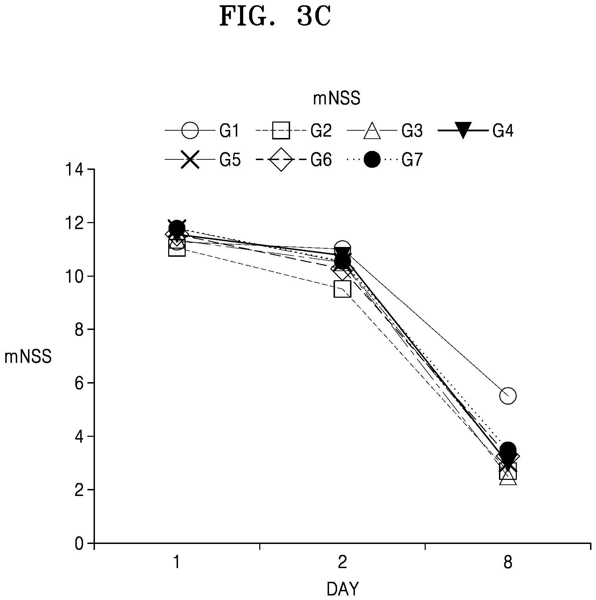

FIG. 3C is a diagram showing the results of measuring mNSS values for the rat model with acute ischemic stroke.

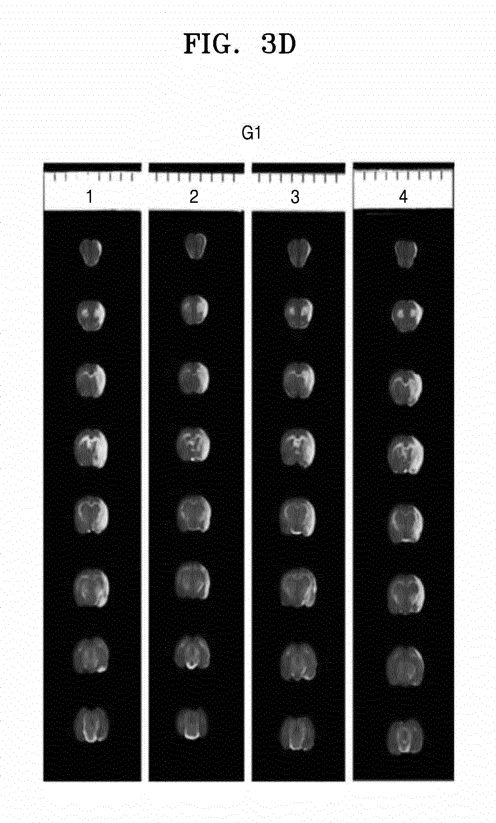

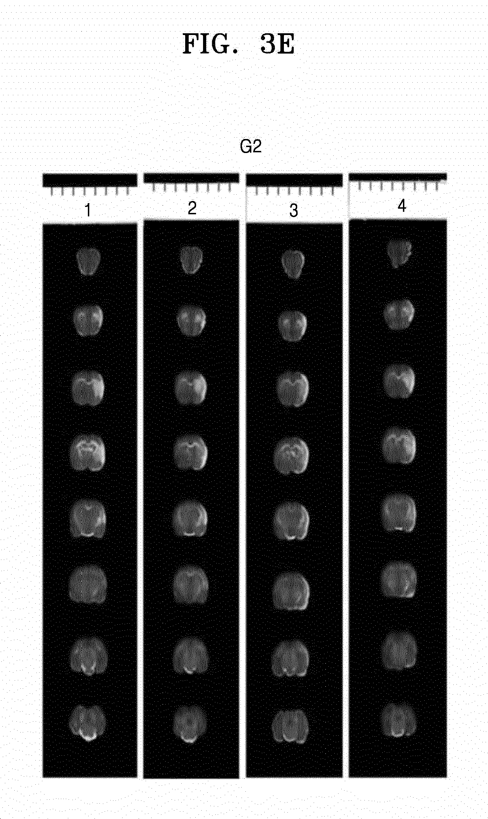

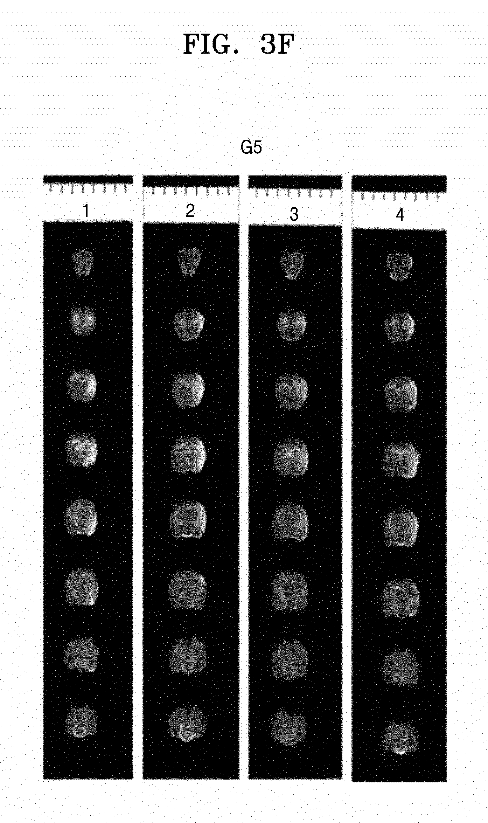

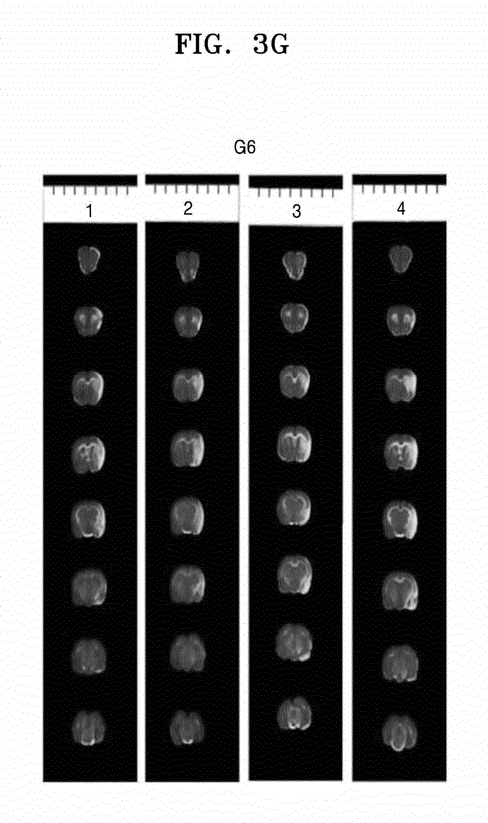

FIGS. 3D, 3E, 3F, and 3G are photographs showing the results of TTC staining on brain matrix slices of brains extracted 7 days after ischemia from the groups G1, G2, G5, and G6 of a rat model with acute ischemic stroke administered with double-stranded RNA. In FIGS. 3D, 3E, 3F, and 3G, from top to bottom, the top slices of the brain matrix to the bottom slices of the brain matrix are shown. As shown in FIGS. 3D, 3E, 3F, and 3G, the cerebral infarction volume was as high as 25 % in the negative control group using PBS as an excipient, whereas the cerebral infarction volume was about 13 %, about 14 %, and about 15 % in the groups G2, G5, and G6, respectively, each treated with the drug of the present disclosure.

As shown in FIGS. 3A to 3G, when the double-stranded RNA having 3'-overhangs was administered in dosages of 20 µg and 50 µg, the therapeutic effect was shown even if the first administration was performed 0.5 hours, 3 hours, 4.5 hours, and 6 hours later.

### 4. Measurement of survival rate

### (1) Production of ischemic stroke animal model - Before surgery

As experimental animals, 72 male Sprague-Dawley rats weighing 240 g to 260 g were used. All surgical tools used in the experiment were autoclaved. The experimental animals were weighed and anesthetized by inhalation (Harvard Apparatus, MA, US) by using a mixture of isoflurane (Hana Pharmaceutical) and air. The anesthesia was induced with 3 % isoflurane and continued with 2 % to 2.5% isoflurane.

### (2) Production of ischemic stroke animal model - Surgery preparation

After confirming that the experimental animal was anesthetized, the forelimbs and incisors were fixed with pins, and it was confirmed whether the neck was fixed in the center of a microscope.

To prevent eye dryness and soreness, eye ointment gel was applied to the eyes. The midline of the neck was marked with a povidone solution to separate the hairs, and the incised skin was made to be as clear as possible.

### (3) Production of ischemic stroke animal model - Surgery

A small vertical incision was made along the midline proximal to the clavicle, and the incision was large enough to fit scissors under the hair to separate the skin. Scissors were inserted into the incision site and carefully opened to separate the hair from the muscle connective tissues. If the soft tissue and muscle were not separated before the muscle incision, muscle damage may occur.

Here, it was carefully handled not to damage the blood vessels in the subcutaneous layer.

After the skin incision (about 6 cm) was made, the left and right salivary glands were separated with fine-tipped scissors, and the right salivary gland was fixed not to move. The connective tissue was delicately separated from the soft tissue surrounding the muscle by using straight forceps. During this process, when the soft tissue was removed by using a cotton swab, the resulting area remained persistent and clear. The sternocleidomastoid (STA) and anterior bronchial muscle were tied to the skin with a 3-0 silk suture, exposing the common carotid artery (CCA) and making a half-knot with a 6-0 silk suture. Then, the vagus nerve, which appeared as a white tip, was carefully separated from the CCA. The branches of the internal carotid artery (ICA) and external carotid artery (ECA) were carefully checked, and mucus and surrounding soft tissue were removed by using a cotton swab.

The superior thyroid artery (STA) branch from the ECA and the ECA were lifted by using a cauterizer to remove the fat underneath the ECA and ICA branch points. When the body fat was completely removed and the pterygopalatine artery (PPA) and MCA branched from the ICA appeared, the probe was guided in the direction of the MCA rather than the direction of the PPA.

### (4) Production of ischemic stroke animal model - Occlusion

A removable tie was made to the CCA by using a 3-0 silk suture to block the blood flow to the ICA and ECA. By combining the CCA, the blood flow from the heart to the brain was prevented. After the STA was cauterized, complete tying was performed with a 3-0 silk suture to the ECA at the upper border over the cauterized area. The reason for tying the ECA near the cauterized STA site is that, when the probe is removed later for reperfusion, the 3-0 silk is pulled by the force of pulling the probe, and there is a risk of death of the animal due to excessive bleeding.

The ICA was then clamped to temporarily block the blood flow to the PPA and ICA. A 3-0 silk suture to be used was placed for stabilization between the CCA and the ECA. An incision was made in the upper half of the ECA between the upper ECA knot and the lower CCA half-knot.

The cut ECA was quickly lifted and the probe (Doccol MCAO suture diameter: 0.37 mm) was carefully inserted. The probe was carefully knotted by using the 3-0 silk suture placed between the CCA and the ECA.

The knot should be tied to the floor of the STA incineration area to prevent bleeding that occurs when the probe is removed. The ICA clamp was removed while slightly pulling the probe and guiding it toward the ICA. The investigation was conducted through the ICA until resistance was felt in the MCA.

When the probe was felt to be inserted toward the MCA, the silicone-coated portion of the probe was gently pushed until it entered the blood vessel. When the silicone-coated portion of the probe entered the blood vessel, minor bleeding may occur. Here, the knot was tighten again to stop the bleeding.

When the probe was determined to reach the MCA, the half-knot in the CCA was untied, the thread used to fasten the animal was removed, and the salivary gland was covered. When adhesion did not occur between the salivary gland and the surrounding tissue, saline was used to fix the salivary gland and tissue together. The skin incision was closed with continuous surgical sutures, and the occlusion was maintained for 90 minutes before the removal of the probe.

### (5) Measurement of survival rate

After the MCAO surgery was performed according to Sections (1) to (4), the rats were divided into four groups, and two groups among them were administered intranasally with 25 µL or 50 µL of 2 µg/µL double-stranded RNA solution a total of 3 times, once at 0.5 hours, once at 3 hours, and once 6 hours. After 7 days, the number of dead and survived rats was counted, and the survival rate was calculated therefrom. The negative control groups were the Sham group, which did not undergo the MCAO surgery and was not administered with the sample, and the MCAO+PBS group, which was administered with PBS after the MCAO surgery. For the MCAO+PBS group, the administration was performed at the same time as the other experimental groups, but PBS was administered instead of double-stranded RNA to animals that underwent the MACO surgery. Then, on the same number of days, the number of dead and survived rats was counted.

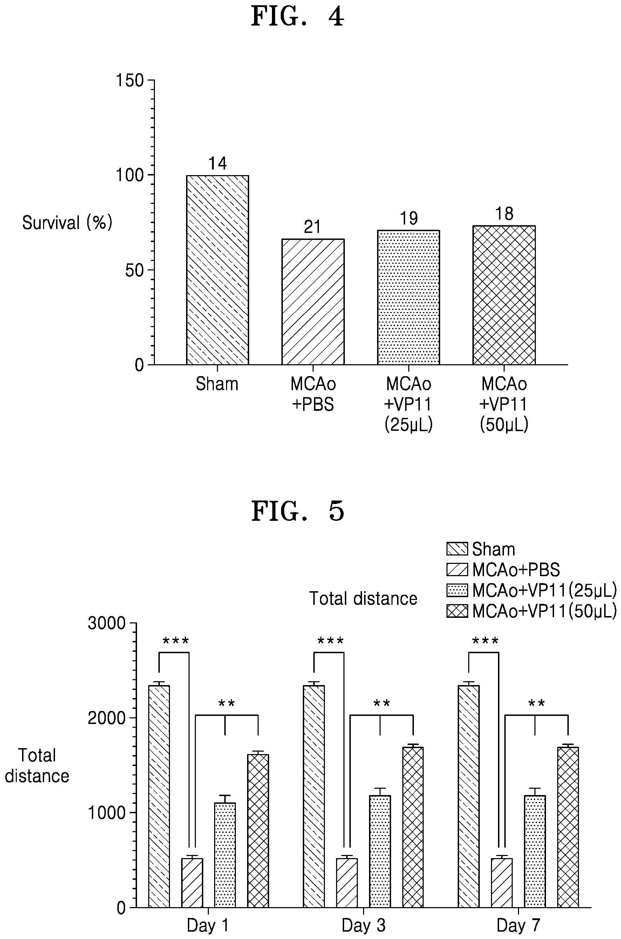

FIG. 4 is a diagram showing the survival rate of the experimental animals according to the dosage of the double-stranded RNA. As shown in FIG. 4, the survival rate of the animals administered with the double-stranded RNA increased in a dosage-dependent manner. In detail, the survival rates for the Sham group, the MCAO+PBS group, the MCAO+VP11 (25 µL) group, and the MCAO+VP11 (50 µL) group were 100 %, 66.67 %, 71.42 %, and 73.68 %, respectively. In FIG. 4, MACO represents a case where middle cerebral artery occlusion surgery was performed, VP11 represents double-stranded RNA, and the numbers above the bars represent the number of animals used.

### 5. Open field test

The rats that underwent the MCAO surgery according to Sections (1) to (4) in "4. Measurement of survival rate" were placed in a testing room of an open field apparatus, and then the total distance travelled was measured.

In detail, the test was conducted in a standard-lit room where a maizer apparatus and a computer capable of running a tracking software can be accommodated. The tracking software used was a video tracking software, SMART, purchased from PanLab/Harvard Apparatus. The movements of rats were recorded and evaluated by using this software. A video camera was attached to the ceiling and floated above the maze so that the camera lens could view the entire maze area. Here, a test administrator paid attention to ensure that the rats in the maze were completely blind. The testing room was cleaned with 95 % ethanol before and after testing each rat.

The rats in the home cage of the rat breeding room were moved to the testing room. The rats were allowed to acclimate to the testing room for at least 30 minutes before starting the test. One rat was removed from the home cage by gently holding its tail and placed in the center of the open field maze. At the same time, the SMART software was activated by pressing a start button to begin tracking the movements of the rat. Rats usually move immediately to the periphery wall of the maze, and the timing of the releasing time of the rats coincides with the tracking capture, and thus the movement at this timing should be recorded. The rat being tested was allowed to move freely and undisturbed through each quadrant of the maze for a 10-minute period. Here, the tracking software recorded the movements. At the end of the testing period, the rat was gently grasped, removed from the maze, and returned to the home cages. This process was repeated for the next rat.

FIG. 5 is a diagram showing the total distance travelled by the rats in the open field test. In FIG. 5, Sham, MCAO+PBS, MCAO+VP11 (25 µL), and MCAO+VP11 (50 µL) refer to, respectively, the Sham group in which no samples were administered to animals that did not undergo the MCAO surgery, the MCAO+PBS group administered with PBS a total of 3 times, once at 0.5 hours, once at 3 hours, and once at 6 hours after the MCAO surgery, and the experimental groups 1 and 2 rats were administered 25 µL or 50 µL of 2 µg/µL double-stranded RNA solution a total of 3 times, once at 0.5 hours, once at 3 hours, and once at 6 hours after the MCAO surgery. In FIG. 5, the horizontal axis represents the number of days since the initial administration of samples, and the vertical axis represents the total distance travelled measured in centimeters (cm).

As shown in FIG. 5, the travel distance of the MCAO+VP11 (25 µL) and the MCAO+VP11 (50 µL) increased in a dosage-dependent manner compared to the control MCAO+PBS.

### 6. Golgi-cox and Sholl analyses

The rats that underwent the MCAO surgery according to Sections (1) to (4) in "4. Measurement of survival rate" were subjected to Golgi-cox and Sholl analyses. In detail, the animal brains were removed and stained by a Golgi-cox method using a FD NeuroTechnologies Rapid GolgiStainTM Kit (FD NeuroTechnologies, Ellicott City, MD, USA) according to the protocol of the manufacturer. The brains were then immersed in a silver impregnation solution for 2 weeks in the dark. Brain tissue sections (thickness of 100 µm to 150 µm) were obtained using a cryostat microtome, and Sholl images of pyramidal neurons for the dendritic arborization studies were processed by using Automated Sholl Analysis of Digitized Neuronal Morphology. The automated Sholl analysis has been previously described by using the NeuronJ imaged plugin, neuron studio visualizer, and custom scripts are described in MATLAB (MathWorks). In this experiment, the Sholl analysis was performed with a 6 µm ring intervals starting from the cell body (soma).

For the rats that underwent the MCAO surgery according to Sections (1) to (4) in "4. Measurement of survival rate", the Golgi-cox and Sholl analyses were performed after 28 days on the Sham group in which no samples were administered to animals that did not undergo the MCAO surgery, the MCAO+PBS group administered with PBS a total of 3 times, once at 0.5 hours, once at 3 hours, and once at 6 hours after the MCAO surgery, and the experimental groups 1 and 2 rats were administered 25 µL or 50 µL of 2 µg/µL double-stranded RNA solution a total of 3 times, once at 0.5 hours, once at 3 hours, and once at 6 hours after the MCAO surgery.

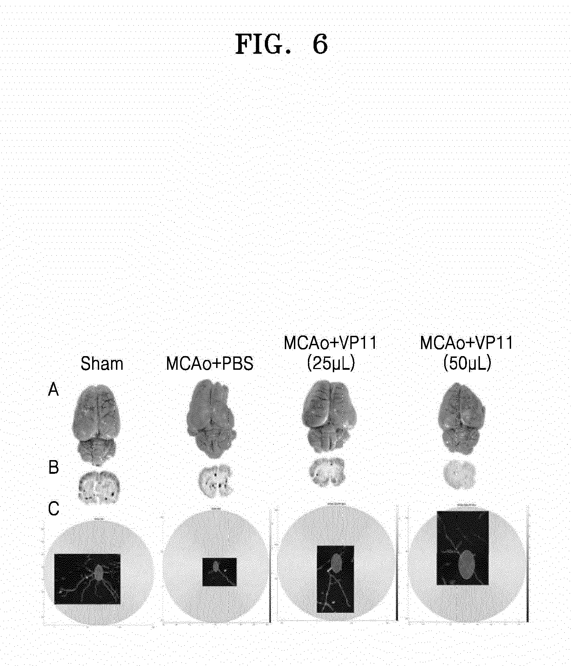

FIG. 6 is a diagram showing results of the Golgi-cox staining and Sholl analyses on the brains of the experimental animals according to a dosage of the double-stranded RNA. FIG. 6 shows micrographs (x400 and x1 ,000) of A) the whole brain 28 days after the surgery, B) vibratome sections of the brain, and C) neurons on the Sholl ring generated by NeuronStudio and MatLab.

As shown in FIG. 6, morphological changes in cortical pyramidal neurons were induced by ischemic damage in the MCAO group, whereas there was no change in the Sham group. In addition, it was confirmed that the dendritic length and spine density were decreased across the cortex in the MCAo+PBS group. To quantify the number of crossing neurites, the Sholl images of pyramidal neurons, with a series of concentric circles extending from 6 µm to 550 µm from the cell body, were analyzed through an automated programs such as NeuronJ plugin, NeuronStudio software, and MATLAB programming. The analysis results of the dendritic branches of the pyramidal neurons showed a complex pattern in the ipsilateral cortex. Compared to the PBS-treated group, the number of crossing neurites of apical dendrites at 30 µm to 150 µm from the cell body of the infarct cortex in the VP11-treated group was significantly increased. In addition, neurite outgrowth was significantly different between the ipsilateral cortex and the sham cortex of the VP11-treated group, but was not in the PBS-treated group.

A significantly high number of branch points and terminal points was observed in the VP11-treated group compared to the PBS-treated group. As a result of the Golgi-cox and Sholl analyses, VP11 (50 µL) showed a higher level of dendritic growth than VP11 (25 µL).

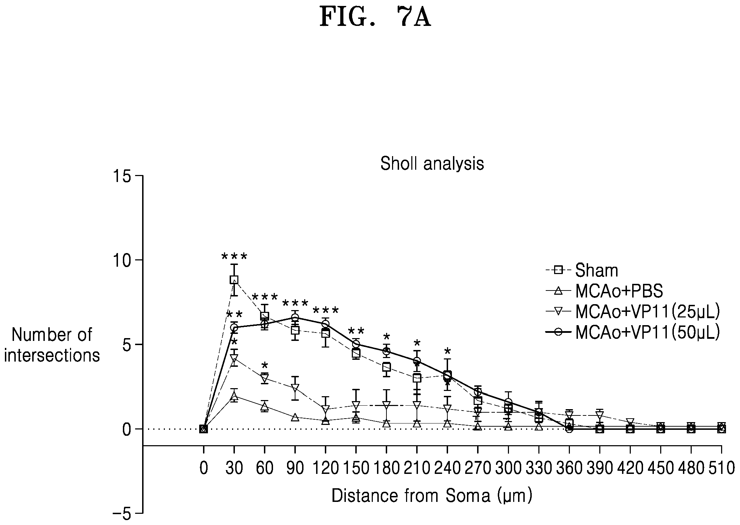

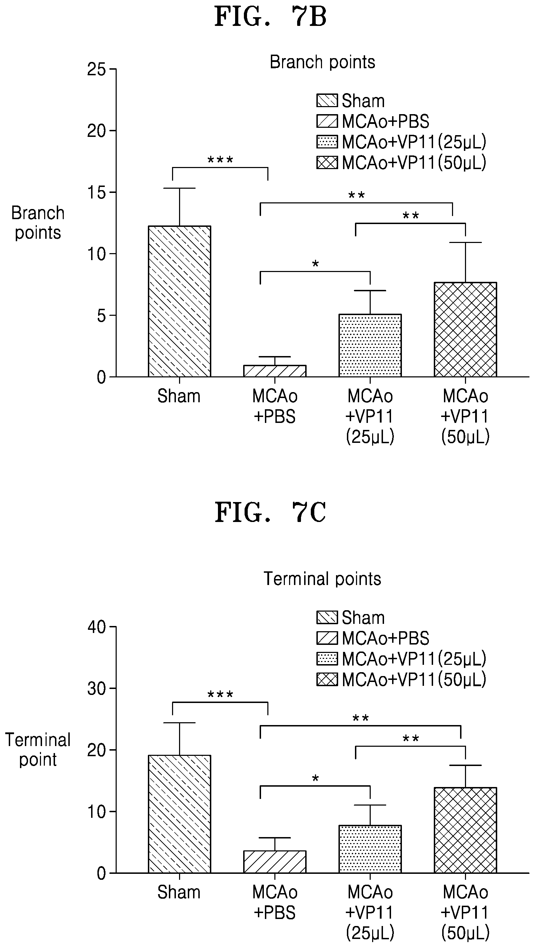

FGIS. 7A, 7B, and 7C are each a diagram showing results of analyzing the neurite outgrowth of pyramidal neurons according to Sholl analysis. FIG. 7A shows that the number of apical dendrites increased in the infarct cortex and contralateral cortex in the VP11-treated group. FIG. 7B shows the branch points, and FIG. 7C shows the terminal points. The VP11 group showed evident changes in the neurite outgrowth, branch points, and terminal points were evident, whereas the PBS group did not.

## Claims

1. A composition for preventing or treating an ischemia-reperfusion injury in a subject, the composition comprising an isolated double-stranded RNA molecule,
wherein the isolated double-stranded RNA molecule comprises: a first strand comprising a 5'-end, a 3'-end, and a region having complementarity with a second strand;
the second strand comprising a 5'-end, a 3'-end, and a region having complementarity with the first strand;
a first overhang comprising one or more consecutive ribonucleotides and being located at the 3'-end of the first strand; and
a second overhang comprising one or more consecutive ribonucleotides and being located at the 3'-end of the second strand.

2. The composition of claim 1, wherein, in the first strand and the second strand, the regions having complementarity independently have a length of 106 nt to 1,648 nt.

3. The composition of claim 1, wherein, in the first strand and the second strand, the first overhang and the second overhang independently have a length of 1 nt to 10 nt.

4. The composition of claim 1, wherein, in the first strand and the second strand, the 5'-ends independently comprise triphosphate, diphosphate, or monophosphate.

5. The composition of claim 1, wherein, in the first strand and the second strand, the 3'-ends independently comprise a hydroxyl group.

6. The composition of claim 1, wherein, in the first strand and the second strand, the regions having complementarity independently is not a homopolynucleotide.

7. The composition of claim 6, wherein the homopolynucleotide is polycytidylate, polyuridylate, polyadenylate, polyinosinylate, or polyguanylate.

8. The composition of claim 1, wherein, in the first strand and the second strand, the first overhang and the second overhang have a same length.

9. The composition of claim 1, wherein, in the first strand and the second strand, the regions having complementarity have 100 % complementarity.

10. The composition of claim 1, wherein, in the first strand and the second strand, the regions having complementarity independently do not comprise a sequence complementary to a nucleotide sequence of a human genome.

11. The composition of claim 1, wherein, in the first strand and the second strand, the regions having complementarity are independently derived from a non-human subject.

12. The composition of claim 1, wherein, in the first strand and the second strand, the first overhang and the second overhang are independently obtained by RNase T1 digestion.

13. The composition of claim 1, wherein, in the first strand and the second strand, the first overhang and the second overhang are independently obtained by hybridizing a pre-first strand with a pre-second strand, the pre-first strand comprising a nucleotide extended at the 3'-end relative to the first strand, and the pre-second strand comprising a nucleotide extended at the 3'-end relative to the second strand, and by contacting a hybridized product with RNase T1 to digest ssRNA at the 3'-end.

14. The composition of claim 1, wherein, in the first strand and the second strand, the first overhang and the second overhang independently have a UAUAG sequence at the 3'-end.

15. The composition of claim 1, wherein, in the first strand and the second strand, the regions having complementarity independently have a UAUAG sequence at the 3'-end.

16. The composition of claim 1, wherein the first strand and the second strand have nucleotide sequences of SEQ ID NOs: 1 and 2, respectively.

17. The composition of claim 1, wherein the composition is for treating brain ischemia, ischemic brain injury, or ischemic stroke (IS).
